## Europäisches Patentamt
## European Patent Office
## Office européen des brevets

(19)

(11) Publication number: **0 197 344**
**B1**

(12) # EUROPEAN PATENT SPECIFICATION

(45) Date of publication of the patent specification:
**03.01.90**

(51) Int. Cl.⁴: **A61F 9/00**

(21) Application number: **86103277.9**

(22) Date of filing: **12.03.86**

(54) **Eye-drop mirror.**

(30) Priority: **14.03.85 DE 8507561 U**

(43) Date of publication of application:
**15.10.86 Bulletin 86/42**

(45) Publication of the grant of the patent:
**03.01.90 Bulletin 90/1**

(84) Designated Contracting States:
**AT BE CH DE FR GB IT LI LU NL SE**

(56) References cited:
**DE-A- 2 020 706**
**DE-U- 8 507 561**
**US-A- 2 382 771**
**US-A- 3 913 575**
**US-A- 4 344 430**

(73) Proprietor: **CHIBRET PHARMAZEUTISCHE GMBH,**
**Charles-de-Gaulle-Strasse 4, D-8000 München 83(DE)**

(72) Inventor: **Schwab, Walter, Dr., Tölzerstrasse 1,**
**D-8038 Gröbenzell(DE)**
Inventor: **Dirscherl, Maria, Dr. med.,**
**Tirschenreutherstrasse 10, D-8000 München 90(DE)**

(74) Representative: **Abitz, Walter, Dr.-Ing. et al, Abitz, Morf,**
**Gritschneder, Freiherr von Wittgenstein**
**Postfach 86 01 09, D-8000 München 86(DE)**

ACTORUM AG

## Description

This invention relates to an eye-drop mirror comprising a mirror and a mounting means for an eye-drop bottle, the mirror surface and the mounting means being at an angle of about 135° to 150° relative to one another, and the center of the mounting means being spaced from the center of the mirror by about 6 centimeters.

An eye-drop mirror of this type is disclosed in US-A 4 344 430. It makes it easier for a patient to self-administer (instil) eye-drops into one eye in that the patient may observe with the contralateral eye in the eye-drop mirror exactly where and how many drops are being administered to the eye. In some indications, such as glaucoma, the exact number of drops is important, so that neither too few nor too many drops should get into the eye. In the eye-drop mirror of US-A 4 344 430 the eye-drop bottle with the medicine to be instilled is adhesively attached at the mounting means. As eye-drop bottles generally are made of breathing plastic material, there exists the danger with that type of attachment that the adhesive would penetrate the bottom of the eye-drop bottle and contaminate the medicine. Further, the mounting is not very secure. US-A 4 344 430 discloses an alternative mounting means to frictionally clamp a medication dispensing container of the squeeze bulb type in an aperture of the mounting means.

US-A 3 913 575 discloses a device for dispensing eye-drops comprising a bottle with a mirror attached to the bottom of the bottle. Due to the small size of the mirror and the relatively large distance between the eye and the mirror, the viewing angle of the mirror is small so that it does not provide much help. This device also makes it necessary to mold the mounting means integral with the wall of the bottle or to adhesively attach it to the bottom of the bottle.

The invention as claimed in claim 1 solves the problem of how to design a eye-drop mirror in which there does not exist the danger of contamination of the medicine through the manner in which the eye-drop bottle is attached to the mounting means, which mirror provides a more secure attachment of the bottle and stays stabile even if the bottle is attached to the eye-drop mirror.

Due to the cup-shaped design of the mounting means, the eye-drop bottle can be securely clamped to the eye-drop mirror. The eye-drop bottle is pressed with its bottom into the cup-shaped mounting means. For the purpose of increasing the clamping effect, a flat rib may be formed at a spot on the inner side of the straight rim of the cup-shaped mounting means.

The eye-drop mirror of this invention usefully comprises a plastic material plate having two surfaces disposed at an angle of about 135° to 150° relative to one another, with the mirror being attached to the one surface while the mounting means is disposed on the other surface. A base (footing) may extend away from the back side of the surface supporting the mirror, said base being flush with the back side of the surface supporting the mounting means. In that way the eye-drop mirror remains standing on the surface carrying the mounting means when it is set down. The eye-drop bottle in that respect stands in upright position, so that no liquid can flow out. Without the base there would be the danger that the eye-drop mirror would tip over on account of the weight of the mirror.

An exemplary embodiment of the invention is described below with reference to the drawings, which shows in:

Figure 1: a perspective view of the eye-drop mirror with an eye-drop bottle securely clamped in position; and in

Figure 2: a sectional view of the eye-drop mirror according to Figure 1.

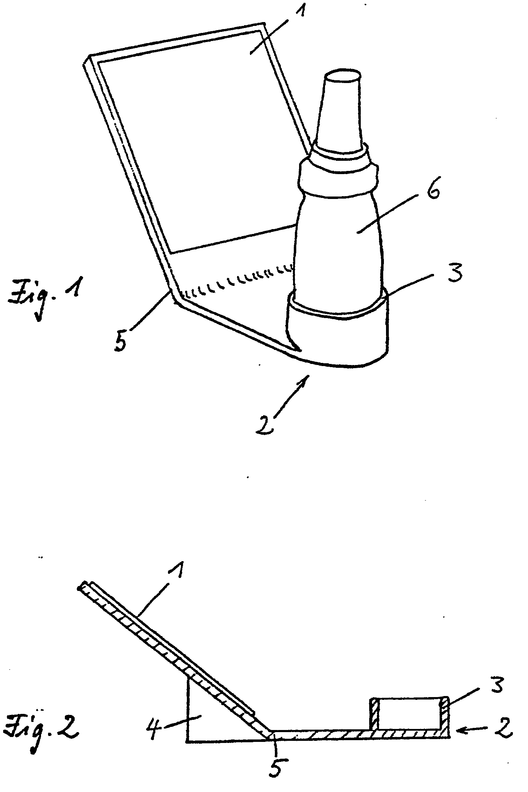

The eye-drop mirror includes an angled plastic material plate 5, to the one surface of which the actual mirror 1 is secured, and at or near the end of the other surface thereof a mounting means 2 is located. The two surfaces are at an angle of about 135 to 150° relative to one another. The mirror 1 is about 4 × 5 centimeters and covers nearly the entire surface of the angled plastic material plate 5.

The center of the mounting means 2 is disposed at a spacing of about 6 centimeters from the center of the mirror 1 at or near the end of the other surface of the angled plastic material plate 5. The mounting means is designed to be cup-shaped with a straight rim 3. The mounting means 2 serves to receive an eye-drop bottle 6. To facilitate insertion of the eye-drop bottle 6, the rim 2 may be somewhat rounded at the upper edge. The inner side of the rim 3 may slightly widen, e.g. by about 1 μm, upwardly. In that way, an improved clamping effect similar to a snap-action is attained when the eye-drop bottle 6 is placed into the mounting means 2.

Underneath the surface supporting the mirror 1 there is disposed a base (footing) 4 serving as a rest, which base is flush with the underside of the other surface supporting the mounting means 2.

The eye-drop mirror according to this invention is suited particularly for use in conjunction with so called breathing eye-drop bottles, such as are required for some medicines for instilling dropwise into the eye. These bottles consist of plastic material, and said material also aids in attaining the snap-type mounting effect. The eye-drop mirror is usefully produced by the injection molding method of plastic material.

## Claims

1. An eye-drop mirror comprising a mirror (1) and a mounting means (2) for an eye-drop bottle (6), the mirror surface and the mounting means being at an angle of about 135° to 150° relative to one another and the center of the mounting means being spaced from the center of the mirror by about 6 centimeters, characterized in that the mounting means (2) is cup-shaped with a straight rim (3) in elevation view, and the mirror and the mounting means are located on the two relatively angled faces of an angled plate (5), there being a base (4) extending underneath the back side of the face on which the mirror is located, said base being flush with the back side of the face on which the mounting means is located so as to serve as a rest.

2. An eye-drop mirror according to Claim 1, wherein the mounting means (2) is of oval plan form.

**Patentansprüche**

1. Augentropfenspiegel mit einem Spiegel (1) und einer Halterung (2) für eine Augentropfenflasche (6), wobei die Spiegeloberfläche und die Halterung miteinander einen Winkel von etwa 135° bis 150° einschließen und die Mitte der Halterung von der Mitte des Spiegels etwa 6 cm entfernt ist, dadurch gekennzeichnet, daß die Halterung (2) schalenförmig mit einem in der Ansicht geradlinigen Rand (3) ausgebildet ist, und der Spiegel und die Halterung auf den beiden zueinander abgewinkelten Flächen einer Winkelplatte (5) angeordnet sind, wobei sich unterhalb der Rückseite der den Spiegel tragenden Plattenfläche eine Basis erstreckt, die mit der Rückseite jener Plattenfläche fluchtet, auf welcher die Halterung angeordnet ist, um als Ständer zu dienen.

2. Augentropfenspiegel nach Anspruch 1, bei dem die Halterung (2) in der Draufsicht oval ist.

**Revendications**

1. Miroir pour gouttes oculaires comprenant un miroir (1) et un moyen de montage (2) pour un flacon à gouttes oculaires (6), la surface du miroir et le moyen de montage faisant un angle d'environ 135 à 150° l'un par rapport à l'autre et le centre du moyen de montage étant espacé du centre du miroir d'environ 6 cm, caractérisé en ce que le moyen de montage (2) est en forme de cuvette comportant une bordure rectiligne (3) quand on l'observe en élévation, et le miroir et le moyen de montage sont disposés sur deux faces inclinées l'une par rapport à l'autre d'une plaque coudée (5), une base 4 s'étendant sous le côté arrière de la face sur laquelle est monté le miroir, ladite base étant en affleurement avec le côté postérieur de la face sur laquelle est disposé le moyen de montage pour servir ainsi de surface de repos.

2. Miroir pour gouttes oculaires selon la revendication 1, dans lequel le moyen de montage (2) est d'une forme ovale quand on l'observe en plan.

Fig. 1

Fig. 2